# EUROPÄISCHE PATENTSCHRIFT

(11) **EP 0 925 276 B1**
(45) Veröffentlichungstag und Bekanntmachung des Hinweises auf die Patenterteilung: **05.12.2001**
(21) Anmeldenummer: 97919014.7
(22) Anmeldetag: 01.09.1997
(51) Int. Cl.: C07C 253/30

(54) **VERFAHREN ZUR HERSTELLUNG VON ALIPHATISCHEN ALPHA, OMEGA-AMINONITRILEN**
PROCESS FOR THE MANUFACTURE OF ALIPHATIC ALPHA, OMEGA AMINO NITRILES
PROCEDE POUR LA PREPARATION D'ALPHA-OMEGA-AMINONITRILES ALIPHATIQUES

(30) Priorität: 10.09.1996 DE 19636767; 11.11.1996 DE 19646436
(43) Veröffentlichungstag der Anmeldung: 30.06.1999
(73) Patentinhaber: BASF AKTIENGESELLSCHAFT, 67056 Ludwigshafen (DE)
(72) Erfinder: EBEL, Klaus, D-68623 Lampertheim (DE); FISCHER, Rolf, D-69121 Heidelberg (DE); FLICK, Klemens, D-76863 Herxheim (DE); MERGER, Martin, D-67227 Frankenthal (DE); VOIT, Guido, D-69198 Schriesheim (DE)
(86) Internationale Anmeldenummer: EP9704733
(87) Internationale Veröffentlichungsnummer: WO9811059

(56) Entgegenhaltungen:
- WO-A-93/16034
- WO-A-96/18603
- DE-A- 4 235 466
- DE-A- 4 446 893

## Beschreibung

Die vorliegende Erfindung betrifft ein Verfahren zur Herstellung von aliphatischen alpha, omega-Aminonitrilen in Gegenwart eines Katalysators.

Aus DE-A 44 468 93 ist ein Verfahren zur Herstellung von aliphatischen alpha,omega-Aminonitrilen durch partielle Hydrierung von aliphatischen alpha,omega-Dinitrilen bei erhöhter Temperatur und erhöhtem Druck in Gegenwart eines Lösungsmittels und eines Katalysators bekannt, indem man einen Katalysator verwendet, der
(a) eine Verbindung auf der Basis eines Metalles, ausgewählt aus der Gruppe, bestehend aus Nickel, Cobalt, Eisen, Ruthenium und Rhodium, enthält und
(b) von 0,01 bis 25, vorzugsweise von 0,1 bis 5 Gew.-%, bezogen auf (a), eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
(c) von 0 bis 5, vorzugsweise von 0,1 bis 3 Gew.-%, bezogen auf (a), einer Verbindung auf der Basis eines Alkalimetalles oder eines Erdalkalimetalles, enthält,
mit der Maßgabe, daß die Komponente (a) nicht auf der Basis von Eisen oder Eisen und einem der Metalle, ausgewählt aus der Gruppe, bestehend aus Cobalt, Ruthenium und Rhodium, besteht, wenn (b) ein Promotor auf der Basis eines Metalles, ausgewählt aus der Gruppe, bestehend aus Titan, Mangan, Chrom und Molybdän, ist sowie mit der weiteren Maßgabe, daß, wenn als Komponente (a) eine Verbindung auf der Basis von nur Ruthenium oder Rhodium oder Ruthenium und Rhodium oder Nickel und Rhodium gewählt wird, der Promotor (b) gewünschtenfalls entfallen kann.

Nachteilig bei diesem Verfahren ist die Bildung von Nebenprodukten, die sich nur sehr schwer von den alpha,omega-Aminonitrilen, wie 6-Aminocapronitril oder gegebenenfalls weiteren Wertprodukten, wie Adipodinitril und Hexamethylendiamin im Falle von 6-Aminocapronitril als Alpha, omega - Aminonitril, abtrennen lassen.

So bilden sich beispielsweise im Falle der Hydrierung von Adipodinitril zu 6-Aminocapronitril und Hexamethylendiamin in wechselnden Mengen unter anderem 1-Amino-2-cyanocyclopenten (ICCP), 2-Aminomethylcyclopentylamin (AMCPA), 1,2-Diaminocyclohexan (DCH) und Bishexamethylentriamin (BHMTA). Aus der US-A 3 696 153 ist bekannt, daß sich AMCPA und DCH nur sehr schwer von Hexamethylendiamin abtrennen lassen.

Ferner ist die Standzeit der Katalysatoren bei diesem Verfahren nicht voll befriedigend.

DE-A-4 235 466 betrifft ein Verfahren zur Herstellung von alpha, omega-Aminonitrilen durch Hydrierung der entsprechenden alpha, omega-Dinitrile in Gegenwart eines Katalysators, der hergestellt worden ist, indem man Eisenschwamm, der bei der Direktreduktion von Eisenerz unterhalb der Schmelztemperatur anfällt, in Form von Granulat oder einer anderen stichigen Form mittels gasförmigen Sauerstoff nicht behandelt oder bei 200 bis 800°C anoxidiert, wobei eine Gewichtszunahme von 0 bis 32 % eintritt, und anschließend mittels gasförmigem Wasserstoff bei 200 bis 500°C reduziert.

Nachteilig bei diesem Verfahren ist, daß die Katalysatoren zu einem erheblichen anteil an Nebenprodukten führen und zudem eine geringe Aktivität aufweisen.

Aufgabe der vorliegenden Erfindung war es daher, ein Verfahren zur Herstellung von aliphatischen alpha,omega-Aminonitrilen durch partielle Hydrierung von aliphatischen alpha,omega-Dinitrilen in Gegenwart eines Katalysators zur Verfügung zu stellen, das die genannten Nachteile nicht aufweist und das die Herstellung von alpha,omega-Aminonitrilen mit hoher Selektivität auf technisch einfache und wirtschaftliche Weise ermöglicht.

Demgemäß wurde ein Verfahren zur Herstellung von aliphatischen alpha,omega-Aminonitrilen durch partielle Hydrierung von aliphatischen alpha,omega-Dinitrilen in Gegenwart eines Katalysators dadurch gekennzeichnet, daß man für die partielle Hydrierung einen Katalysator verwendet, der
(a) Eisen oder eine Verbindung auf der Basis von Eisen oder deren Gemische enthält und
(b) von 0,01 bis 5 Gew.-% bezogen auf (a) eines Promotors auf der Basis von 2,3,4 oder 5 Elementen ausgewählt aus der Gruppe bestehend aus Aluminium, Silizium, Zirkonium, Titan und Vanadium sowie
(c) von 0 bis 5 Gew.-% bezogen auf (a) einer Verbindung auf der Basis eines Alkali- oder Erdalkalimetalls, enthält,
gefunden.

Bevorzugte Katalysator-Vorläufer sind solche, in denen die Komponente (a) zu 90 bis 100 Gew.-%, 95 bis 100 Gew.-%, bevorzugt 92 bis 99 Gew.-%, 96 bis 99 Gew.-% und insbesondere 98 bis 99 Gew.-% bezogen auf (a) Eisenoxide, Eisenhydroxide, Eisenoxyhydroxide oder deren Gemische enthält. Als solche kommen beispielsweise Eisen-(III)-oxid, Eisen-(II,III)-oxid, Eisen-(II)-oxid, Eisen(II)-hydroxid, Eisen-(III)-hydroxid oder Eisenoxydhydroxid wie FeOOH in Betracht. Verwendet werden können synthetisch hergestellte oder natürlich vorkommende Eisenoxide, Eisenhydroxide oder Eisenoxyhydroxide, wie Magneteisenstein (Magnetit), der im Idealfall mit Fe₃O₄ beschrieben werden kann, Brauneisenstein, der im Idealfall mit Fe₂O₃ · H₂O beschrieben werden kann, oder Roteisenstein (Hämatit), der im Idealfall mit Fe₂O₃ beschrieben werden kann.

Bevorzugte Katalysator-Vorläufer sind weiterhin solche, in denen Komponente (b) von 0,01 bis 5 Gew.-%, vorzugsweise 0,5 bis 4 Gew.-%, insbesondere 1 bis 3 Gew.-% eines Promotors auf der Basis von 2,3,4 oder 5 Elementen ausgewählt aus der Gruppe bestehend aus Aluminium, Zirkonium, Silizium, Titan und Vanadium, wie Aluminium, Silizium und Vanadium enthält.

Bevorzugte Katalysator-Vorläufer sind weiterhin solche, in denen Komponente (c) von 0 bis 5 Gew.-%, 0 bis 0,5 Gew.-%, vorzugsweise 0,05 bis 0,4 Gew.-%, 0,1 bis 3 Gew.-%, insbesondere 0,1 bis 0,2 Gew.-% einer Verbindung auf der Basis eines Alkali- oder Erdalkalimetalls, vorzugsweise ausgewählt aus der Gruppe bestehend aus Lithium, Natrium, Kalium, Rubidium, Cäsium, Magnesium und Calcium enthält.

Bei den erfindungsgemäßen Katalysatoren kann es sich um Voll-oder Trägerkatalysatoren handeln. Als Trägermaterialien kommen beispielsweise poröse Oxide wie Aluminiumoxid, Siliziumdioxid, Alumosilikate, Lanthanoxid, Titandioxid, Zirkondioxid, Magnesiumoxid, Zinkoxid, und Zeolithe sowie Aktivkohle oder Mischungen davon in Betracht.

Die Herstellung erfolgt in der Regel derart, daß man Vorläufer der Komponente (a) gewünschtenfalls zusammen mit Vorläufern der Promotoren Komponenten (b) und gewünschtenfalls mit Vorläufern der Spurenkomponenten (c) in Gegenwart oder Abwesenheit von Trägermaterialien (je nachdem welcher Katalysatortyp gewünscht ist) ausfällt, gewünschtenfalls den so erhaltenen Katalysator-vorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b) und gewünschtenfalls (c) tränkt, wobei man die einzelnen Komponenten gleichzeitig oder nacheinander zugeben kann, oder indem man die Komponenten (a), gewünschtenfalls (b) und (c) auf den Träger nach an sich bekannten Methoden aufsprüht.

Als Vorläufer der Komponenten (a) kommen in der Regel gut wasserlösliche Salze des Eisens wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle und Halbmetalle wie Nitrate, Chloride, Acetate, Formiate und Sulfate in Betracht, vorzugsweise Nitrate.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man üblicherweise bei Temperaturen im Bereich von 150 bis 500, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 200 bis 500, vorzugsweise von 250 bis 400°C 2 bis 24 h einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 1 pro 1 Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80, vorzugsweise von 25 bis 35°C mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500, vorzugsweise von 200 bis 350°C in einer Wasserstoff-haltigen Atmosphäre vor.

Die Katalysatoren können als Festbettkatalysatoren in Sumpf-oder Rieselfahrweise oder als Suspensionskatalysatoren eingesetzt werden.

Als Ausgangsstoffe im erfindungsgemäßen Verfahren werden aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile I vorzugsweise in Gegenwart eines Lösungsmittels unter Verwendung eines Katalysators partiell zu alpha,omega-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 30, vorzugsweise von 3 bis 30, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 40, vorzugsweise von 3 bis 30 MPa wählt. Bevorzugt führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 2,0, vorzugsweise von 0,3 bis 1,0 kg Adipodinitril/1*h. Auch hier kann man durch Veränderung der Verweilzeit den Umsatz und damit die Selektivität gezielt einstellen.

Die partielle Hydrierung kann man in einem üblichen hierfür geeigneten Reaktor (R1 in Zeichnung) durchführen.

Bei der Hydrierung erhält man eine Mischung, die 6-Aminocapronitril, Hexamethylendiamin und Adipodinitril enthält.

Die Abtrennung von 6-Aminocapronitril, Hexamethylendiamin und einem im wesentlichen Adiponitril enthaltenden Teil von der Mischung kann in an sich bekannter Weise, vorzugsweise destillativ, beispielsweise gemäß der DE-A 195 002 22 oder der Deutschen Anmeldung 19 548 289.1, gleichzeitig oder nacheinander erfolgen.

Die Destillation in der ersten Kolonne K1 in der Zeichnung) führt man dabei so durch, daß man die Mischung, enthaltend im wesentlichen 6-Aminocapronitril, Hexamethylendiamin, Ammoniak, Adipodinitril und Hexamethylenimin, bevorzugt eine Mischung, enthaltend im wesentlichen von 1 bis 70, vorzugsweise von 5 bis 40 Gew.-% 6-Aminocapronitril,
von 1 bis 70, vorzugsweise von 5 bis 40 Gew.-% Adipodinitril,
von 0,1 bis 70, vorzugsweise von 1 bis 40 Gew.-% Hexamethylendiamin,
von 0,01 bis 10, vorzugsweise von 0,05 bis 5 Gew.-% Hexamethylenimin und
von 5 bis 95, vorzugsweise von 20 bis 85 Gew.-% Ammoniak, in der Regel in einer üblichen Destillationskolonne, bei einer Sumpftemperatur im Bereich von 60 bis 250, vorzugsweise von 100 bis 200°C und einem Druck im Bereich von 5 bis 30, vorzugsweise von 12 bis 25 bar in Gegenwart von einer oder mehreren unter den Destillationsbedingungen inerten Verbindungen A durchführt, die bei einem Druck von 18 bar bei einer Temperatur im Bereich von 60 bis 220°C sieden, unter Erhalt von Ammoniak als Kopfprodukt und eines Sumpfes I, wobei man den Ammoniak nicht vollständig abtrennt.

Als Verbindung A kommen Substanzen in Betracht, die unter den Destillationsbedingungen inert sind und einen Siedepunkt im Bereich von 60 bis 250, vorzugsweise von 60 bis 150°C bei einem Druck von 18 bar aufweisen. Beispielhaft seien genannt: Alkane, Cycloalkane, Aromaten, Naphthene, Alkohole, Ether, Nitrile und Amine mit den zuvor genannten Eigenschaften, insbesondere C₅-C₈-Alkane und C₂-C₄-Alkanole, besonders bevorzugt n-Pentan, Cyclohexan, Triethylamin, Ethanol, Acetonitril, n-Hexan, Di-n-propylether, Isopropanol, n-Butylamin, Benzol, ganz besonders bevorzugt Ethanol.

Üblicherweise gibt man Verbindung A in einer Menge im Bereich von 0,1 bis 50, vorzugsweise von 1 bis 10 Gew.-%, bezogen auf den Sumpf I, zu.

Man unterwirft den Sumpf I, enthaltend im wesentlichen 6-Aminocapronitril, Hexamethylendiamin, Adipodinitril, Hexamethylenimin, inerte Verbindung(en) A sowie Ammoniak, wobei der Ammoniak-Gehalt geringer ist gegenüber dem Reaktoraustrag aus R1 einer zweiten Destillation unter Erhalt einer Mischung aus der(n) inerten Verbindung(en) A und Ammoniak als Kopfprodukt und eines Sumpfes II, wobei man die Destillation bei einer Sumpftemperatur im Bereich von 100 bis 250, vorzugsweise von 140 bis 200°C, und einem Druck im Bereich von 2 bis 15, vorzugsweise von 4 bis 12 bar durchführt, mit der Maßgabe, daß man die Drücke der ersten und der zweiten Kolonne (K2 in der Zeichnung) so aufeinander abstimmt, daß man bei einer jeweiligen Sumpftemperatur von maximal 250°C eine Kopftemperatur von über 20°C erhält. Es kann auch vorteilhaft sein, daß man die Kondensation am Kopf der zweiten Kolonne bei tieferen Temperaturen durchführt, wobei der Kopfabzug, der aus reinem oder höher konzentriertem Ammoniak besteht, in die erste Kolonne zurückgeführt wird, oder
den Kopfabzug der zweiten Kolonne dampfförmig nach Druckerhöhung mit einem Verdichter in die erste Kolonne oder in deren Kondensator zurückführt.

Man unterwirft den Sumpf II, enthaltend im wesentlichen 6-Aminocapronitril, Hexamethylendiamin, Adipodinitril, Hexamethylenimin und inerte Verbindung(en) A in einer dritten Kolonne (K3 in der Zeichnung) einer Destillation unter Erhalt der inerten Verbindung(en) A als Kopfprodukt und eines Sumpfes III, wobei man die Destillation bei einer Sumpftemperatur im Bereich von 50 bis 250, vorzugsweise von 140 bis 200°C und einem Druck im Bereich von 0,05 bis 2, vorzugsweise von 0,2 bis 1 bar durchführt, mit der Maßgabe, daß man die als Kopfprodukt erhaltene(n) inerte(n) Verbindung(en) A der zweiten Kolonne zuführt, und gewünschtenfalls die Destillation in Gegenwart von einer oder mehreren unter den Destillationsbedingungen inerten Verbindung(en) B durchführt, die bei einem gegebenen Druck von 0,3 bar im Bereich von 20 bis 250, vorzugsweise von 60 bis 170°C sieden.

### Als Verbindung B seien beispielhaft genannt:

Alkane, Cycloalkane, Aromaten, Naphthene, Alkohole, Ether, Nitrile und Amine mit den zuvor genannten Eigenschaften, insbesondere Di-n-butylether, Valeronitril, n-Octan, Cyclooctan, n-Hexylamin, Hexamethylenimin, Hexamethylendiamin bevorzugt Hexamethylenimin und/oder Hexamethylendiamin, besonders bevorzugt Hexamethylenimin.

In einer bevorzugten Ausführungsform wählt man als Verbindung B Hexamethylenimin und/oder Hexamethylendiamin, oder, besonders bevorzugt, man gibt keine weitere Verbindung B zu.

Bevorzugt führt man Verbindung B der Kolonne K3 in einer Menge im Bereich von 0,01 bis 50, vorzugsweise von 0,5 bis 10 Gew.-%, bezogen auf den Sumpf II, zu.

Man unterwirft den Sumpf III, enthaltend im wesentlichen 6-Aminocapronitril, Hexamethylendiamin, Adipodinitril, Hexamethylenimin und gewünschtenfalls inerte Verbindung(en) B, in einer vierten Kolonne (K4 in der Zeichnung) einer Destillation unter Erhalt eines Kopfproduktes KP1, enthaltend im wesentlichen Hexamethylenimin, gewünschtenfalls inerte Verbindung(en) B und eines Seitenabzuges SA1, enthaltend im wesentlichen Hexamethylendiamin, wobei die Sumpftemperatur der Kolonne im Bereich von 50 bis 250°C und der Druck im Bereich von 0,05 bis 1,5 bar liegen unter Erhalt eines Sumpfes IV.

Gewünschtenfalls stattet man die Kolonne mit einer Trennwand im Bereich zwischen Zulauf und Seitenabzug aus (Petlyuk-Kolonne), so daß das gewonnene Hexamethylendiamin im wesentlichen frei ist von Hexamethylenimin und inerten Verbindung(en) B sowie von anderen Leichtsiedern, wobei
man Kopfprodukt KP1 und/oder HMD aus dem Seitenabzug SA1 bei Bedarf der dritten Kolonne zuführt oder gegebenenfalls nur teilweise der dritten Kolonne zuführt und den Rest ausschleust.

Man unterwirft den Sumpf IV, enthaltend im wesentlichen 6-Aminocapronitril und Adiponitril sowie eventuelle Hochsieder in einer fünften Kolonne (K5 in der Zeichnung) einer Destillation, unter Erhalt von 6-Aminocapronitril mit einer Reinheit von mindestens 95 %, vorzugsweise 99 bis 99,9 %, als Kopfprodukt und eines Seitenabzuges V, bestehend im wesentlichen aus Adipodinitril, sowie eines Sumpfes V, der aus Hochsiedern und geringen Mengen Adipodinitril besteht.

Gewünschtenfalls stattet man die Kolonne mit einer Trennwand im Bereich zwischen Zulauf und Seitenabzug aus, so daß das gewonnene Adipodinitril niedrigere Anteile an Hochsiedern enthält, wobei die Destillation bei einer Sumpftemperatur im Bereich von 50 bis 250°C und einem Druck im Bereich von 10 bis 300 mbar durchgeführt wird.

Anstatt das Adiponitril als Seitenabzug V zu erhalten, kann man auch den Sumpf V der Kolonne K5, enthaltend Adipodinitril und höhersiedenden Verbindungen, in einer weiteren Kolonne K6 destillativ auftrennen und dabei Adipodinitril als Kopfprodukt VI erhalten.

Erfindungsgemäß behandelt man den im wesentlichen Adipodinitril enthaltenden Teil, der bei der beschriebenen destillativen Aufarbeitung des bei der Hydrierung von Adipodinitril anfallenden Reaktionsgemisches als Seitenabzug. V der Kolonne k5, als Kopfprodukt VI der Kolonne K6 oder als Sumpfprodukt der Kolonne D5, vorzugsweise als Seitenabzug V der Kolonne D5 anfällt, mit einer Säure oder einem sauren Ionentauscher.

Als Säuren oder saure Ionentauscher kommen in erster Linie Substanzen in Betracht, die gegenüber primären, sekundären und tertiären gesättigten und ungesättigten Aminen wie Enaminen als Protonendonatoren fungieren können. Besonders geeignet sind dazu Säuren mit einem p_{Ka}-Wert von höchstens 10, vorzugsweise höchstens 7.

Als Säuren können anorganische Säuren wie Salpetersäure, vorzugsweise Schwefelsäure insbesondere als 100 Gew.-%ige Schwefelsäure oder als eine mindestens 90 Gew.-%, vorzugsweise 96 Gew.-%, enthaltende Mischung insbesondere mit Wasser oder Phosphorsäure, organische Säuren, beispielsweise Carbonsäuren wie Adipinsäure, 2-Ethylhexansäure, Pimelinsäure, Korksäure, Undecandisäure, Terephthalsäure, Cyclohexancarbonsäure, beispielsweise Sulfonsäure wie p-Toluolsulfonsäure, Benzolsulfonsäure, als saure Ionentauscher beispielsweise Lewatit S100G1, Amberlyst 15, Dowex 50 WX 8, Bay. Kat. K 2431, Amberlite IR-120 sowie Gemische solcher Säuren und sauren Ionentauscher eingesetzt werden.

Die Umsetzung des Adiponitril mit der Säure kann in Gegenwart eines flüssigen Verdünnungsmittels wie Wasser erfolgen, wobei das flüssige Verdünnungsmittel zusammen mit der Säure oder vor oder nach der Säurezugabe zu dem Adiponitril gegeben werden kann.

Die direkte Behandlung des von höhersiedenden Verbindungen nicht befreiten Adiponitrils, beispielsweise das Sumpfprodukt V der Kolonne K5, wenn diese keinen Adipodinitril-Seitenabzug enthält, ist ebenso möglich. In diesem Fall erhöht sich der Verbrauch an Säure oder sauren Ionentauscher und die Menge des anfallenden Rückstandes nach der Abtrennung des Adipodinitril.

Das Molverhältnis der Säuregruppen zu den im Rückstand vorhandenen basischen Verbindungen sollte mindestens äquimolar, vorzugsweise überäquimolar sein. Als vorteilhaft haben sich Säurezugaben von 0,01 bis 10 Gew.-%, insbesondere 0,1 bis 2 Gew.-% bezogen auf Adipodinitril, erwiesen.

Die Umsetzung des Adiponitrils mit der Säure kann in an sich bekannter Weise, wie durch Mischen oder durch Leiten des Adiponitrils über ein Ionentauscher-Festbett, vorteilhaft bei Temperaturen von 2 bis 250°C, insbesondere 30 bis 100°C erfolgen, wodurch sich Reaktionszeiten von 1 Sekunde bis 30 Minuten, insbesondere 1 Sekunde bis 10 Minuten ergeben.

Aus der Mischung kann das Adipodinitril in an sich bekannter Weise, vorteilhaft destillativ oder extraktiv, abgetrennt werden.

Bei Zugabe eines flüssigen Verdünnungsmittel wie Wasser bei der Umsetzung des Rückstandes mit der Säure kann man das flüssige Verdünnungsmittel vor der Abtrennung des Adipodinitrils vorzugsweise adsorptiv, insbesondere destillativ abtrennen.

Ebenso können die nach der Säurezugabe erhaltenen Umsetzungsprodukte und gegebenenfalls überschüssige Säure vorteilhaft durch Extraktion, beispielsweise mit Wasser, von Adipodinitril abgetrennt werden.

Das nach dem erfindungsgemäßen Verfahren erhaltene Adipodinitril kann erneut für die partielle Hydrierung zu Hexamethylendiamin und 6-Aminocapronitril verwendet werden, wobei eine Aufpegelung von Nebenprodukten vermieden wird, die eine spezifikationsgerechte Herstellung von Hexamethylendiamin und/oder 6-Aminocapronitril verhindern und/oder die Standzeit des Katalysators für die partielle Hydrierung negativ beeinflussen.

Nach dem erfindungsgemäßen Verfahren erhält man alpha,omega-Aminonitrile in guten Selektivitäten und mit geringeren Mengen an Nebenprodukten als nach dem Stand der Technik. Des weiteren weisen die erfindungsgemäß eingesetzten Katalysatoren ein deutlich längere Standzeit auf als vergleichbare Katalysatoren aus dem Stand der Technik. Die alpha,omega-Aminonitrile sind wichtige Ausgangsverbindungen zur Herstellung von cyclischen Lactamen, insbesondere 6-Aminocapronitril für Caprolactam.

In den Beispielen bedeuten:
- ADN =: Adipodinitril
- ACN =: 6-Aminocapronitril
- HMD =: Hexamethylendiamin
- DCH =: cis + trans-1,2-Diaminocyclohexan
- AMCPA =: 1-Amino-2-aminomethylcyclopentan
- BHMTA =: Bis-Hexamethylentriamin
- ICCP =: 1-Amino-2-cyanocyclopenten

### Beispiel 1

### a) Katalysatorherstellung

Durch Tempern eines Gemischs aus Magnetit, Kaliumcarbonat, Al₂O₃, Calciumcarbonat, Brechen der erstarrten Schmelze und Sieben gemäß A.B. Stiles, T.A. Koch, Catalyst Manufacture (1995) S. 167/68 wurde eine oxidische Masse folgender Zusammensetzung erhalten: 1,1 Gew.-% K₂O, 3,0 Gew.-% Al₂O₃, 2,3 Gew.-% CaO, 0,11 Gew.-% Si, 0,01 Gew.-% Ti, Rest Fe-Oxide.

Anschließend wurde diese Masse im N₂/H₂-Strom bei 450°C 72 h reduziert, bei Raumtemperatur mit einem N₂/Luft-Gemisch (24 h mit 1 % Luft in Stickstoff), wobei die Temperatur im Katalysatorbett nicht über 45°C stieg, passiviert und mit Wasser 7 Tage gewaschen.

Die erhaltene Katalysatormasse hatte folgende Zusammensetzung: 1,2 Gew.-% Al, 0,74 Gew.-% Ca, 0,02 Gew.-% K, 0,11 Gew.-% Si, 0,01 Gew.-% Ti, Rest Fe/Fe-Oxid. Die Summe der Promotoren aus Gruppe b) liegt bei 1,32 Gew.-%, die Summe der Promotoren aus Gruppe c) gerechnet als Oxide, liegt bei 1,06 Gew.-%.

### b) Partielle Hydrierung von ADN zu ACN

Ein Rohrreaktor (Länge 180 cm, d = 30 mm) wurde mit 740 ml (1819 g) der nach (a) hergestellten Katalysatormasse befüllt und drucklos im Wasserstoffstrom (500 Nl/h) reduziert. Dabei wurde die Temperatur innerhalb von 24 h von 30°C auf 340°C angehoben und anschließend 72 h bei 340°C gehalten.
Nach Absenken der Temperatur wurde dem Reaktor bei 250 bar ein Gemisch aus 400 ml/h ADN, 1140 ml/h NH₃ und 500 Nl/h H₂ zugeführt. Nach einer Laufzeit von 9700 h bei 110°C betrug der ADN-Umsatz 45 %, die ACN-Selektivität 42 % und die HMD-Selektivität 57 %. Ferner wurden 3700 ppm DCH, 55 ppm AMCPA, 30 ppm ICCP und 1560 ppm BHMTA gefunden.

### Beispiel 2

### a) Katalysatorherstellung

Der Katalysator wurde hergestellt durch sechsstündiges Tempern eines Magnetiterzes bei 1500°C unter Stickstoff. Das verwendete Magnetiterz hatte folgende Zusammensetzung:
72 Gew.-% Fe, 0,07 Gew.-% Al, 0,03 Gew.-% Ca, 0,04 Gew.-% Mg, 0,11 Gew.-% Si, 0,01 Gew.-% Ti, Rest Sauerstoff. Die Summe der Promotoren aus Gruppe b) liegt bei 0,19 Gew.-%, die Summe der Promotoren aus Gruppe c) gerechnet als Oxide, liegt bei 1,03 Gew.-%.

Der abgekühlte Schmelzblock wurde im Backenbrecher zerkleinert und eine Siebfraktion der Teilchengröße 1,5 bis 3 mm ausgesiebt. Der oxidische Katalysator wurde im H₂/N₂-Strom bei 450°C 72 Stunden lang reduziert. Nach Abkühlen unter Stickstoff auf Raumtemperatur wurde der Fe-Katalysator mit einem N₂/Luft-Strom passiviert (24 h mit 1 % Luft in Stickstoff), wobei darauf geachtet wurde, daß die Temperatur im Katalysatorbett nicht über 45°C anstieg.

### b) Partielle Hydrierung von ADN zu ACN

Drei in Reihe geschaltete Rohrreaktoren (Gesamtlänge 4,5 m, d = 6 mm) wurden mit 141 ml (239 g) des nach Beispiel 1 a) hergestellten Katalysators (1,5 - 3 mm Splitt) befüllt und anschließend drucklos im Wasserstoffstrom (200 1/h) reduziert. Hierzu wurde die Temperatur innerhalb von 24 Stunden von 70°C auf 340°C angehoben und anschließend 72 Stunden bei 340°C gehalten. Nach Absenken der Temperatur wurde dem Reaktor bei 250 bar ein Gemisch aus 75,0 ml/h ADN, 365 ml/h NH3 und 200 Nl/h H2 zugeführt. Der Versuch wurde 950 Stunden lang betrieben. Eine Abnahme der Katalysator-Aktivität und der ACN-Ausbeute und Selektivität wurde nicht beobachtet. Unter den genannten Bedingungen wurden in Abhängigkeit von der Temperatur folgende Ergebnisse erhalten (Tabelle 1) :

**Tabelle 1**

| Temperatur [°C] | ADN-Umsatz [%] | ACN- Selektivität [%] | HMD-Selektivität [%] | ACN-+ HMD-Selektivität [%] | DCH | AMCPA | BHMTA | ICCP |
|---|---|---|---|---|---|---|---|---|
| | | | | | [ppm, bez. auf HMD] | | | |
| 80 | 47,3 | 80,4 | 18,5 | 98,9 | 3700 | 430 | | 80 |
| 90 | 72,1 | 67,3 | 31,7 | 99,0 | 2900 | 250 | | 48 |
| 96 | 89,4 | 48,8 | 50,2 | 99,0 | 2100 | 120 | 990 | 60 |
| 107 | 99,9 | 0,6 | 98,6 | 99,2 | 1200 | 25 | | 53 |

Die Ergebnisse in Tab. 1 zeigen, daß in Gegenwart der erfindungsgemäßen Katalysatoren eine gezielte Einstellung des ACN/HMD-Verhältnisses möglich ist und daß ACN- + HMD-Selektivitäten von rund 99 % erreicht werden. Sie demonstrieren weiterhin, daß die Menge an DCH und AMCPA, bezogen auf Hexamethylendiamin, vom ADN-Umsatz abhängt. Je mehr HMD erzeugt wird, desto weniger DCH und AMCPA (bezogen auf HMD) liegen vor.

Aus U.S. 4.282.381, Spalte 2, Tabelle 1, ist bekannt, welche Mengen an Nebenprodukten bei der Hydrierung von Adipodinitril zu Hexamethylendiamin in Gegenwart von Eisen-Katalysatoren durchschnittlich entstehen. Für DCH sind dies 2400 - 4000 ppm, für AMCPA 100 - 300 ppm und für BHMTA 3000 - 5000 ppm.

Es war nicht vorherzüsehen und damit überraschend, daß bei der Herstellung von ACN/HMD-Gemischen in Gegenwart der erfindungsgemäßen Eisen-Katalysatoren (siehe z. B. Tab. 1, ACN-Selektivität 48,8 %) nicht größere, sondern kleinere Mengen an DCH und AMCPA und BHMTA gebildet werden.

### Beispiel 3

### a) Katalysatorherstellung

Der Katalysator wurde hergestellt durch Tempern (6 Stunden bei 1500°C unter Stickstoff) eines Gemisches eines Magnetiterzes, das mit 3,4 Gew.-% Al₂O₃ innig vermischt wurde. Das verwendete Magnetiterz hatte folgende Zusammensetzung:
72 Gew.-% Fe, 0,07 Gew.-% Al, 0,03 Gew.-% Ca, 0,04 Gew.-% Mg, 0,11 Gew.-% Si, 0,01 Gew.-% Ti, Rest Sauerstoff.

Der abgekühlte Schmelzblock wurde im Backenbrecher zerkleinert und eine Siebfraktion der Teilchengröße 1,5 bis 3 mm ausgesiebt. Die Analyse des so erhaltenen Katalysatorvorläufers ergab folgende Zusammensetzung:
69,6 Gew.-% Fe, 1,81 Gew.-% Al, 0,029 Gew.-% Ca, 0,038 Gew.-% Mg, 0,11 Gew.-% Si, 0,01 Gew.-% Ti, Rest Sauerstoff. Die Summe der Promotoren aus Gruppe b) liegt bei 1,93 Gew.-%, die Summe der Promotoren aus Gruppe c), gerechnet als Oxide, liegt bei 1,04 Gew.-%.

Der oxidische Katalysator wurde im H₂/N₂-Strom bei 450°C 72 Stunden lang reduziert. Nach Abkühlen unter Stickstoff auf Raumtemperatur wurde der Fe-Katalysator mit einem N₂/Luft-Strom passiviert (24 h mit 1 % Luft in Stickstoff), wobei darauf geachtet wurde, daß die Temperatur im Katalysatorbett nicht über 45°C anstieg.

### b) Partielle Hydrierung von ADN zu ACN

Die Hydrierung von Adipodinitril wurde mit dem in Beispiel 3 a) beschriebenen Eisenkatalysator durchgeführt. Die Bedingungen der Katalysator-Aktivierung und die pro Stunde zugefahrenen Mengen an ADN, Ammoniak und Wasserstoff entsprachen Beispiel 2 b).

In Tabelle 2 sind die bei einer Hydriertemperatur von 97°C erhaltenen Werte für ADN-Umsatz, ACN- und HMD-Selektivität und die Mengen an Nebenkomponenten enthalten.

**Tabelle 2**

| Temperatur [°C] | ADN-Umsatz [%] | ACN-Selektivität [%] | HMD-Selektivität [%] | ACN-+ HMD-Selektivität [%] | ACH | AMCPA | BHMTA | ICCP |
|---|---|---|---|---|---|---|---|---|
| | | | | | [ppm, bez. auf HMD] | | | |
| 97 | 91,8 | 46,4 | 52,9 | 99,3 | 1900 | 90 | 900 | 55 |

## Patentansprüche

1. Verfahren zur Herstellung von aliphatischen alpha, omega-Aminonitrilen durch partielle Hydrierung von aliphatischen alpha,omega-Dinitrilen in Gegenwart eines Katalysators **dadurch gekennzeichnet, daß** man für die partielle Hydrierung einen Katalysator verwendet, der
(a) Eisen oder eine Verbindung auf der Basis von Eisen oder deren Gemische enthält und
(b) von 0,01 bis 5 Gew.-% bezogen auf (a) eines Promotors auf der Basis von 2,3,4 oder 5 Elementen ausgewählt aus der Gruppe bestehend aus Aluminium, Silizium, Zirkonium, Titan und Vanadium sowie
(c) von 0 bis 5 Gew.-% bezogen auf (a) einer Verbindung auf der Basis eines Alkali- oder Erdalkalimetalls, enthält.

2. Verfahren nach Anspruch 1, wobei man als Verbindung auf der Basis von Eisen ein Eisenoxid oder Gemische von Eisenoxiden einsetzt.

3. Verfahren nach Anspruch 1 oder 2, wobei man einem Promotor auf der Basis Aluminium, Silizium und Vanadium einsetzt.

4. Verfahren nach den Ansprüchen 1 bis 3, wobei der Katalysator ein Trägerkatalysator ist.

5. Verfahren nach den Ansprüchen 1 bis 3, wobei der Katalysator ein Vollkatalysator ist.

6. Verfahren nach den Ansprüchen 1 bis 5, wobei man als Dinitril Adipodinitril einsetzt unter Erhalt von 6-Aminocapronitril.

7. Verfahren nach den Ansprüchen 1 bis 6 zur gleichzeitigen Herstellung von 6-Aminocapronitril und Hexamethylendiamin ausgehend von Adipodinitril durch
(1) partielle Hydrierung von Adipodinitril in Gegenwart eines Katalysators unter Erhalt einer Mischung enthaltend 6-Aminocapronitril, Hexamethylendiamin und Adipodinitril und
(2) Abtrennung von 6-Aminocapronitril und Hexamethylendiamin aus der Mischung.

8. Verfahren nach Anspruch 7, wobei man dem im verbleibenden, wesentlichen Adipodinitril enthaltenden Teil 0,01 bis 10 Gew.-% einer Säure oder eines sauren Ionentauschers, bezogen auf Adipodinitril, zusetzt und das Adipodinitril von dem Gemisch abtrennt.

9. Verfahren nach Anspruch 7 oder 8, **dadurch gekennzeichnet, daß** man das Adipodinitril von dem Gemisch destillativ abtrennt und eine Säure einsetzt, deren Siedepunkt unter dem für die Destillation gewählten Druck über dem Siedepunkt von Adipodinitril liegt.

10. Verfahren nach den Ansprüchen 7 bis 9, **dadurch gekennzeichnet. daß** man eine Säure mit einem pKₐ-Wert von höchstens 10 einsetzt.

11. Verfahren nach den Ansprüchen 7 bis 10, **dadurch gekennzeichnet, daß** man das nach den Verfahren erhaltene Adipodinitril erneut zur gleichzeitigen Herstellung von 6-Aminocapronitril und Hexamethylendiamin ausgehend von Adipodinitril einsetzt.

12. Verfahren nach den Ansprüchen 1 bis 11, **dadurch gekennzeichnet, daß** man die Hydrierung in einer Suspension vornimmt.

13. Verfahren nach den Ansprüchen 1 bis 11, **dadurch gekennzeichnet, daß** man die Hydrierung in einem Festbettreaktor vornimmt.

## Claims

1. A process for preparing aliphatic alpha,omega-amino nitriles by partial hydrogenation of aliphatic alpha,omega-dinitriles in the presence of a catalyst, wherein the catalyst used for the partial hydrogenation comprises
(a) iron or a compound based on iron or mixtures thereof and
(b) from 0.01 to 5% by weight, based on (a), of a promoter based on 2,3,4 or 5 elements selected from the group consisting of aluminum, silicon, zirconium, titanium and vanadium and
(c) from 0 to 5% by weight, based on (a), of a compound based on an alkali metal or alkaline earth metal.

2. A process as claimed in claim 1, wherein an iron oxide or mixtures of iron oxides is employed as compound based on iron.

3. A process as claimed in claim 1 or 2, wherein a promoter based on aluminum, silicon and vanadium is employed.

4. A process as claimed in any of claims 1 to 3, wherein the catalyst is a supported catalyst.

5. A process as claimed in any of claims 1 to 3, wherein the catalyst is an unsupported catalyst.

6. A process as claimed in any of claims 1 to 5, wherein adiponitrile is employed as dinitrile, and 6-aminocapronitrile is obtained.

7. A process as claimed in any of claims 1 to 6 for the simultaneous preparation of 6-aminocapronitrile and hexamethylenediamine starting from adiponitrile by
(1) partial hydrogenation of adiponitrile in the presence of a catalyst to obtain a mixture comprising 6-aminocapronitrile, hexamethylenediamine and adiponitrile and
(2) removal of 6-aminocapronitrile and hexamethylenediamine from the mixture.

8. A process as claimed in claim 7, wherein from 0.01 to 10% by weight of an acid or an acidic ion exchanger, based on adiponitrile, are added to the remaining part which essentially comprises adiponitrile, and the adiponitrile is removed from the mixture.

9. A process as claimed in claim 7 or 8, wherein the adiponitrile is removed from the mixture by distillation, and an acid whose boiling point is above the boiling point of adiponitrile under the pressure chosen for the distillation is employed.

10. A process as claimed in any of claims 7 to 9, wherein an acid with a pKₐ not exceeding 10 is employed.

11. A process as claimed in any of claims 7 to 10, wherein the adiponitrile obtained after the process is employed anew for the simultaneous preparation of 6-aminocapronitrile and hexamethylenediamine starting from adiponitrile.

12. A process as claimed in any of claims 1 to 11, wherein the hydrogenation is carried out in a suspension.

13. A process as claimed in any of claims 1 to 11, wherein the hydrogenation is carried out in a fixed bed reactor.

## Revendications

1. Procédé de préparation d'alpha, oméga-aminonitriles aliphatiques par hydrogénation partielle d'alpha, oméga-dinitriles aliphatiques en présence d'un catalyseur, **caractérisé en ce que** l'un utilise pour l'hydrogénation partielle un catalyseur contenant
(a) du fer ou un composé à base de fer, ou leurs mélanges, et
(b) de 0,01 à 5% en poids, par rapport à (a), d'un promoteur à base de 2, 3, 4 ou 5 éléments choisis dans le groupe formé par l'aluminium, le silicium, le zirconium, le titane et le vanadium, ainsi que
(c) de 0 à 5% en poids, par rapport à (a), d'un composé à base d'un métal alcalin ou alcalino-terreux.

2. Procédé selon la revendication 1, où l'on met en oeuvre, en tant que composé à base de fer, un oxyde de fer ou des mélanges d'oxydes de fer.

3. Procédé selon la revendication 1 ou 2, où l'on met en oeuvre un promoteur à base d'aluminium, de silicium et de vanadium.

4. Procédé selon les revendications 1 à 3, où le catalyseur est un catalyseur supporté.

5. Procédé selon les revendications 1 à 3, où le catalyseur est un catalyseur plein.

6. Procédé selon les revendications 1 à 5, où l'on met en oeuvre, en tant que dinitrile, de l'adiponitrile avec obtention de 6-aminocapronitrile.

7. Procédé selon les revendications 1 à 6 pour la préparation simultanée de 6-aminocapronitrile et d'hexaméthylènediamine à partir d'adiponitrile, par:
(1) hydrogénation partielle d'adiponitrile en présence d'un catalyseur, avec obtention d'un mélange contenant du 6-aminocapronitrile, de l'hexaméthylènediamine et de l'adiponitrile, et
(2) séparation du 6-aminocapronitrile et de l'hexaméthylènediamine du mélange.

8. Procédé selon la revendication 7, où l'on ajoute à la partie restante contenant essentiellement de l'adiponitrile de 0,01 à 10% en poids d'un acide ou d'un échangeur d'ions acide, par rapport à l'adiponitrile, et l'on isole l'adiponitrile du mélange.

9. Procédé selon la revendication 7 ou 8, **caractérisé en ce que** l'on isole l'adiponitrile du mélange par distillation, et l'on met en oeuvre un acide dont le point d'ébullition à la pression choisie pour la distillation est supérieur au point d'ébullition de l'adiponitrile.

10. Procédé selon les revendications 7 à 9, **caractérisé en ce que** l'on met en oeuvre un acide d'un pKₐ d'un maximum de 10.

11. Procédé selon les revendications 7 à 10, **caractérisé en ce que** l'on remet en oeuvre l'adiponitrile obtenu par le procédé pour la préparation simultanée de 6-aminocapronitrile et d'hexaméthylènediamine.

12. Procédé selon les revendications 1 à 11, **caractérisé en ce que** l'on entreprend l'hydrogénation dans une suspension.

13. Procédé selon les revendications 1 à 11, **caractérisé en ce que** l'on entreprend l'hydrogénation dans un réacteur à lit fixe.
